(19)

Europäisches
Patentamt
European
Patent Office
Office européen
des brevets

(11) **EP 3 501 381 A1**

(12) **EUROPEAN PATENT APPLICATION**

(43) Date of publication:
**26.06.2019 Bulletin 2019/26**

(51) Int Cl.:
*A61B 5/00* *(2006.01)*      *A61B 5/024* *(2006.01)*

(21) Application number: **17210372.3**

(22) Date of filing: **22.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicants:
• **Stichting IMEC Nederland**
  **5656 AE  Eindhoven (NL)**
• **IMEC vzw**
  **3001 Leuven (BE)**

(72) Inventors:
• ZHANG, Yifan
  3001 Leuven (BE)
• GROENENDAAL, Willemijn
  3001 Leuven (BE)
• SONG, Shuang
  3001 Leuven (BE)
• VULLINGS, Rik
  3001 Leuven (BE)

(74) Representative: **AWA Sweden AB**
**P.O. Box 5117**
**200 71 Malmö (SE)**

(54) **A METHOD AND A SYSTEM FOR TIME DOMAIN SIGNAL RECONSTRUCTION FOR REPRESENTING HEART ACTIVITY**

(57)      A method for time domain signal reconstruction for representing heart activity of a subject from a photoplethysmogram (PPG) signal comprises: receiving (402) a PPG signal carrying information of heart activity of the subject; decomposing (404) frequency information of the PPG signal into a plurality of components, each component having a frequency spectrum and a weight; for each component of the plurality of components: comparing (406) the frequency spectrum of the component to a spectrum mask based on an estimate of heart rate of the subject; and adjusting (408) the weight of the component based on the comparing; and reconstructing (410) a time domain signal based on recombination of the plurality of components with adjusted weights.

*Fig. 4*

EP 3 501 381 A1

**Description**

<u>Technical field</u>

The present inventive concept relates to a method and a system for time domain signal reconstruction for representing heart activity of a subject. In particular, the present inventive concept relates to time domain signal reconstruction of a photoplethysmogram (PPG) signal for reducing motion artifacts from the PPG signal.

<u>Background</u>

Acquisition of signals representing a heart activity of a subject is important or of interest in many contexts. The signals may be used in clinical settings to provide information for treatment of the subject, but may also be used for general monitoring of a physical condition of the subject. Also, monitoring of heart activity may be of interest to the subject, e.g. for monitoring exercise or other activities of the subject. Photoplethysmography is a technology of interest in monitoring heart activity as it may be provided in a wearable device which may minimally affect a comfort of the subject wearing the device.

However, a photoplethysmogram (PPG) signal may be affected by motion artifacts. Thus, if the subject is moving, motion artifacts may be generated which may cause quality of an acquired PPG signal to deteriorate or may prevent making any assessments based on the PPG signal. This is accentuated, when the PPG signal is acquired during daily life of a subject. Thus, for long-term monitoring of heart activity using a PPG signal, motion artifact reduction is needed.

Further, motion artifact reduction is often performed in frequency domain in order to extract e.g. heart rate or other frequency information from the PPG signal. However, frequency domain information may not represent variations in heart rate within an analyzed time interval. Thus, if heart rate variability, or even a peak-to-peak duration, is to be determined, a time domain representation of the PPG signal after motion artifact reduction may need to be determined.

The time domain representation may be even more sensitive to motion artifacts, since it may not only be desired to determine average frequencies. Therefore, a robust method for time domain signal reconstruction is desired in order to enable analysis of heart activity on a short time scale, such as within a single heartbeat.

<u>Summary</u>

An objective of the present inventive concept is to provide an improved method and system for time domain signal reconstruction for representing a heart activity of a subject from a PPG signal.

This and other objectives of the invention are at least partly met by the invention as defined in the independent claims. Preferred embodiments are set out in the dependent claims.

According to a first aspect, there is provided a method for time domain signal reconstruction for representing heart activity of a subject from a photoplethysmogram, PPG, signal, said method comprising: receiving a PPG signal carrying information of heart activity of the subject; decomposing frequency information of the PPG signal into a plurality of components, each component having a frequency spectrum and a weight; for each component of the plurality of components: comparing the frequency spectrum of the component to a spectrum mask based on an estimate of heart rate of the subject; and adjusting the weight of the component based on the comparing; and reconstructing a time domain signal based on recombination of the plurality of components with adjusted weights.

According to the invention, relevant frequency information in the PPG signal is defined in two different ways. Firstly, by using a spectrum mask based on the estimated heart rate. Secondly, by decomposing the frequency information into a plurality of components having different weights. Thanks to combining the frequency information determined in these two ways, a more reliable and robust manner of forming a time domain signal representation of the PPG signal, while reducing influence of noise or motion artifacts in the time domain representation.

The estimated heart rate may not be entirely correct and may be based on a relatively long time frame of the PPG signal, e.g. longer than the time frame for which the time domain signal reconstruction is to be performed (and may thus not be exactly correct for a time frame of the PPG signal).

It should be realized that the estimate of the heart rate may be determined in any manner, such as based on the received PPG signal, or based on another signal, which may roughly determine heart rate.

Since the estimated heart rate may not be exactly accurate in representing the heart rate, the spectrum mask may define an allowable spectrum of frequencies around the estimated heart rate. Further, by using the spectrum mask on components formed by decomposing the frequency information, the spectrum mask is not directly applied to the PPG signal and small errors in the determination of the estimated heart rate will not severely affect the possibility to still provide an accurate time domain signal reconstruction of the PPG signal.

Further, by adjusting the weights of the components determined by decomposing frequency information, the frequency decomposition is improved by information of an estimated heart rate, which allows the reconstruction of the time domain signal to be improved.

Hence, thanks to the invention, a time domain signal reconstruction of a PPG signal may be formed, which may allow detailed analysis of a PPG signal in order to extract information on a level of a single heartbeat.

It should be realized that the decomposing of frequency information of the PPG signal may be performed in a time segment of the signal. Thus, the PPG signal may be divided into a plurality of sequential time segments. The decomposing of frequency information of the PPG signal may be performed for each time segment, such that a separate set of time-series components is formed for each time segment. Hence, the time domain signal reconstruction may be formed for each time segment based on the corresponding set of time-series components, whereas a longer representation in the time domain signal reconstruction may be formed by stitching time domain signal reconstructions for sequential time segments to each other.

According to an embodiment, the method further comprises determining the spectrum mask based on the estimate of heart rate of the subject.

Hence, the spectrum mask may be determined as part of the forming of a reconstruction of a time domain signal. This implies that the method may be autonomous in determining the information needed in order to reconstruct the time domain signal.

However, it should be realized that the spectrum mask may be determined by a different process, e.g. if a rough estimate of the heart rate is determined based on another signal. Then, the spectrum mask may be received, e.g. from another unit or another process of estimating the heart rate, and may be used in reconstruction of the time domain signal.

According to an embodiment, the spectrum mask comprises normal distributions around the estimate of the heart rate and one or more harmonics of the estimate of the heart rate, the spectrum mask representing probabilities of frequencies corresponding to heart rate information of the PPG signal based on the normal distributions.

This implies that a probability of a frequency in a signal representing heart activity is based on a normal distribution around the estimate of the heart rate. Hence, an error in the estimate of the heart rate is allowed, while the probability of frequency information not exactly corresponding to the estimate is decreasing according to the normal distribution.

The frequency information of the PPG signal may comprise harmonics of the heart rate and, therefore, the spectrum mask may also allow frequency information corresponding to harmonics to be maintained. This may improve time domain signal reconstruction.

The spectrum mask may comprise the second and third harmonics of the estimate of the heart rate. The heart rate of subjects may vary within a known frequency interval, e.g. 0.4-4 Hz. This implies that a pre-processing of the signal may include a bandpass filter to remove any irrelevant frequencies. Hence, only second and third harmonics may need to be included in the spectrum mask, as higher order harmonics may anyway be outside a frequency range specified by the bandpass filter.

It should be realized that other types of distributions around the estimate of the heart rate may be allowed. For instance, the spectrum mask could be formed as another type of continuous probability distribution. Also, the spectrum mask could be formed as a function defining a probability of "1" for a frequency range around the estimate of the heart rate and a frequency range around each of one or more harmonics, and defining a probability of "0" for any other frequency.

According to an embodiment, the comparing comprises correlating the frequency spectrum of the component with the spectrum mask representing probabilities of frequencies corresponding to heart rate information of the PPG signal for scaling the weight of the component.

This implies that a weight of a component may be adjusted using the correlation of the frequency spectrum of the component with the spectrum mask. Hence, if the frequency spectrum comprises few or no frequencies within the frequencies defined by the spectrum mask, the weight will be low, whereas if the frequency spectrum is strong within the frequencies defined by the spectrum mask, the weight will be high. Then, in reconstruction of the time domain signal, the components representing frequencies which correspond to the heart rate will provide a large contribution, such that noise and motion artifacts in different frequencies may be suppressed.

According to an embodiment, the decomposing frequency information of the PPG signal is performed for a plurality of sequential time segments to form a plurality of time-series components, wherein the decomposing frequency information of the PPG signal into the plurality of time-series components comprises performing singular spectrum analysis (SSA) of the PPG signal.

The PPG signal may be formed as a sequence of time segments, wherein each time segment may be separately reconstructed. The spectrum mask may be different for each time segment. Alternatively, the spectrum mask may be determined based on an estimate of the heart rate which applies to or is determined based on a plurality of time segments. Then, a common spectrum mask may be used for a plurality of time segments in a sequence.

SSA may decompose frequency information in a time-series analysis of a signal. SSA may thus be particularly useful for decomposing frequency information of a PPG signal divided in a plurality of sequential time segments. The components formed by SSA will be given weights based on their contribution to the full PPG signal. These weights may then be adjusted based on the correlation with the spectrum mask.

According to an embodiment, the method further comprises calculating a Fourier transform of a component for determining the frequency spectrum of the component.

Thus, once the component has been established in decomposition of frequency information, the frequency spectrum may need to be separately determined for the component.

It should be realized that different types of frequency analysis may be performed. For instance, a Fourier transform, such as by calculating a fast Fourier transform (FFT) may be used. However, other types of frequency analysis may alternatively be used.

According to an embodiment, the method further comprises performing motion artifact reduction on the PPG signal to form a cleaned PPG signal, wherein decomposing frequency information of the PPG signal into the plurality of components is based on the cleaned PPG signal.

This implies that separate motion artifact reduction may first be performed on the PPG signal before the reconstruction of the time domain signal is applied. Hence, the method for time domain signal reconstruction may provide a suitable complement in forming a time domain signal for a PPG signal which has already been subject to motion artifact reduction.

The received PPG signal may thus be in time domain (as a cleaned or raw PPG signal). However, the received PPG signal may alternatively be in frequency domain, possibly after motion artifact reduction has been performed. As yet another alternative, the received PPG signal may be in time-frequency domain, wherein a time-varying frequency spectrum of the PPG signal is provided. Again, the time-frequency domain representation of the received PPG signal may or may not have been subject to motion artifact reduction.

According to an embodiment, the method further comprises determining the estimate of heart rate based on the received PPG signal. Thus, the PPG signal may also be used for determining the estimate of the heart rate, and there is no need to include another sensor for determining the estimate of the heart rate. This implies that an algorithm for determining the time domain signal reconstruction may be based solely on the PPG signal and does not necessarily need any additional input.

According to an embodiment, the method further comprises dividing the received PPG signal into sequential time segments, wherein the decomposing, comparing, adjusting and reconstructing is performed on the PPG signal within a time segment.

This implies that a relatively short time period may be used for each time segment, such that the time domain signal may be reconstructed with high quality within each time segment. Hence, a motion artifact or other noise occurring need only affect a short time period. Further, only a short time period need be included in forming the frequency domain information such that only fast variations of the heart activity will affect the time domain signal representation.

According to an embodiment, each time segment may be 4 seconds long. This implies that at least two heart beats should occur within a single time segment. However, it should be understood that other lengths of the time segment may be used. For instance, the time period of a time segment may be in the range of 2-8 seconds. A short time period may imply that it is difficult to extract information from the PPG signal, as the signal may in some instances only include a single heartbeat. On the other hand, a long time period may imply that the heart activity may vary within the time segment and also increases a likelihood of motion artifacts occurring within the time segment.

According to an embodiment, the method further comprises analyzing a reconstructed time domain signal spanning more than a single time segment for reducing noise in the reconstructed time domain signal.

The analysis may allow removing any noise, such as discontinuations in the reconstructed time domain signal, that may be generated in stitching of signals for sequential time segments to each other.

Further, if the reconstructed time domain signal within a time segment is still affected by noise or motion artifact, the analysis of a time domain signal spanning more than a single time segment may allow using a clean time segment adjacent to the affected time segment in order to aid in at least extracting a heart rate from the affected time segment.

According to a second aspect, the time domain signal reconstruction formed by the first aspect is used for monitoring heart rate variability.

Effects and features of this second aspect are largely analogous to those described above in connection with the first aspect. Embodiments mentioned in relation to the first aspect are largely compatible with the second aspect.

Thus, the reconstructed time domain signal may be of sufficient quality in order to allow a PPG signal to be used, not only for an estimation of heart rate, but for monitoring variations in heart rate. In some embodiments, the duration of each single heart beat may be determined. This may allow monitoring health of a subject in a more detailed manner enabling extraction of further parameters relating to the health of the subject.

It should be realized that the reconstructed time domain signal may also be used in other manners. For instance, the reconstructed time domain signal may be used for improving an accuracy of an estimate of the heart rate, which may be used as input to the spectrum mask utilized in the method of the first aspect.

According to a third aspect, there is provided a computer program product or a computer-readable medium storing computer-readable instructions such that when executed on a processing unit the computer program product will cause the processing unit to perform the method according to the first aspect.

Effects and features of this third aspect are largely analogous to those described above in connection with the first, and second aspects. Embodiments mentioned in relation to the first, and second aspects are largely compatible with the third aspect.

The computer-readable instructions, which may be stored in the form of a computer program product or on a computer-readable medium, may implement an algorithm for performing time domain signal reconstruction. The computer-readable instructions may thus allow a processing unit to be set up, by receiving the computer program product or the computer-readable medium, for performing the method. Hence, the computer program product or computer-readable medium may be delivered to a processing unit at any point in time in order to configure the processing unit for allowing the method to be performed.

According to a fourth aspect, there is provided a system for time domain signal reconstruction for representing heart activity of a subject from a photoplethysmogram (PPG) signal, said system comprising: a processing unit, said processing unit being configured to: receive a PPG signal carrying information of heart activity of the subject; decompose frequency information of the PPG signal into a plurality of components, each component having a frequency spectrum and a weight; for each component of the plurality of components: compare the frequency spectrum of the component to a spectrum mask based on an estimate of heart rate of the subject; and adjust the weight of the component based on the comparing; and reconstruct a time domain signal based on recombination of the plurality of components with adjusted weights.

The system allows a time domain signal to be reconstructed. The processing unit may be arranged in a suitable unit for providing the reconstructed time domain signal where needed. For instance, the processing unit may be integrated with a wearable device for providing a reconstructed time domain signal in a device worn by the subject. This may imply that analysis based on the reconstructed time domain signal may be quickly provided directly to the subject via an interface, such as a display, of the wearable device.

According to an embodiment, the system further comprises a PPG detector for generating the PPG signal. Thus, the system may generate the PPG signal and may further process the PPG signal in order to provide a high quality time domain signal reconstruction.

According to an embodiment, the PPG detector may comprise a PPG light source configured for emitting green light towards a skin surface of the subject and a PPG light sensor arranged to detect reflected green light.

Using green light, a PPG signal representing a pulsatile component of blood flow may be acquired, while the PPG signal is relatively insensitive to motion artifacts as light penetrates a relatively small distance into tissue. Therefore, a PPG detector comprising a green light source and a sensor arranged to detect reflected green light may be particularly useful.

According to an embodiment, the system further comprises a motion detector for generating a motion reference signal for use in motion artifact reduction of the PPG signal. Thus, the system may also comprise a motion detector such that motion artifact reduction may be performed based on the motion reference signal. The PPG signal received for determining of the time domain signal reconstruction may be a cleaned PPG signal after motion artifact reduction.

According to an embodiment, the motion detector comprises a motion reference light source configured for emitting infrared light towards a skin surface of the subject and a motion reference light sensor arranged to detect reflected infrared light.

Using infrared light, a reflectance-type PPG may be acquired which is sensitive to motion artifacts. The motion artifacts may affect the motion reference signal in a similar way as the PPG signal generated by the PPG detector will be affected. Therefore, a motion detector comprising an infrared light source and a sensor arranged to detect reflected infrared light may be particularly useful.

According to an embodiment, the system is a wearable device. Thus, the system may be worn by a subject allowing long-term monitoring of heart activity.

According to an embodiment, the system comprises a common housing, in which PPG detector, motion detector and processing unit may be arranged. This implies that the system may be arranged in a single unit and may allow acquiring signals and also process the signals for providing a time domain signal reconstruction.

The common housing may comprise an element allowing the housing to be worn by the subject so as to provide the system in the form of a wearable device. However, it should be realized that the system may form a wearable device in other ways, such as comprising a plurality of housings, which are each arranged to be wearable by the subject.

In one embodiment, the common housing may comprise an adhesive patch for attachments to a skin surface of the subject. The adhesive patch may provide the PPG detector and the motion detector in close relation to skin of the subject so as to enable acquiring of signals of high quality.

In other embodiments, the common housing may comprise a band or belt which may be applied around a body part, such as a wristband, for arranging the common housing on a subject. This may enable the PPG detector and the motion detector to be worn by the subject without affecting comfort of the subject.

The processing unit may be further configured to analyze the time domain signal reconstruction in order to extract information, such as heart rate and/or heart rate variability. Such extracted information may also be output to a display of the common housing, such that the subject may directly view information of heart activity on the display.

However, the processing unit may be arranged in a different unit remote from the common housing. Thus, the common housing may be provided with a communication unit, for wired or wireless communication, and may transmit, possibly pre-processed, PPG signals and motion reference signals to an external unit housing the processing unit. The communication may be over a computer network, such as the Internet, which may also allow the processing unit to be arranged on any server or computer connected to the network and may hence be said to be provided "in the cloud".

Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.

Fig. 1 is a schematic view of a system for acquiring and processing of a PPG signal.
Fig. 2a-b are schematic view illustrating acquiring of a reflectance-type PPG signal and penetration of green and red light, respectively, in tissue.
Fig. 3 is a flow chart illustrating a method for motion artifact reduction.
Fig. 4 is a flow chart illustrating a method for time domain signal reconstruction.
Fig. 5 is a flow chart illustrating processing steps of combined motion artifact reduction and time domain signal reconstruction.
Fig. 6 is a graph illustrating a classifier for deciding between a motion or no motion situation.
Fig. 7 is a graph illustrating subtraction of a motion reference time-frequency representation from a PPG time-frequency representation.
Fig. 8a is a graph illustrating a frequency spectrum mask and Fig. 8b is a graph illustrating adjusted weights of SSA components based on the frequency spectrum mask.
Fig. 9 are graphs illustrating an original PPG signal and a reconstructed time domain signal.
Fig. 10 are graphs illustrating an original PPG signal, a motion reference signal, a reconstructed time domain signal and a de-noised signal.

Detailed description

Ambulatory monitoring for health status/indicators of a subject requires high reliability in data collection and processing during various activities of daily life, like walking, jogging and stretching. However, during these activities, a photoplethysmogram (PPG) signal is susceptible to corruption by motion artifacts, which may originate from sensor movement, tissue deformation, and ambient light changing, etc.

Thus, if a PPG signal is to be used for long-term monitoring of heart activity of a subject, motion artifact reduction is an important issue to handle. Further, a time domain signal reconstruction of a clean, or at least relatively clean, PPG signal may allow extracting features on a short time scale, such as relating to a single heart beat or a few heart beats. This implies that heart rate variability may be extracted.

Both motion artifact reduction and time domain signal reconstruction may be applied to an acquired PPG signal. Therefore, although the present application is mainly related to time domain signal reconstruction, in the following description, motion artifact reduction will also be described. It should be realized that motion artifact reduction need not necessarily be performed before the time domain signal reconstruction or that motion artifact reduction may be performed in other manners than those described below.

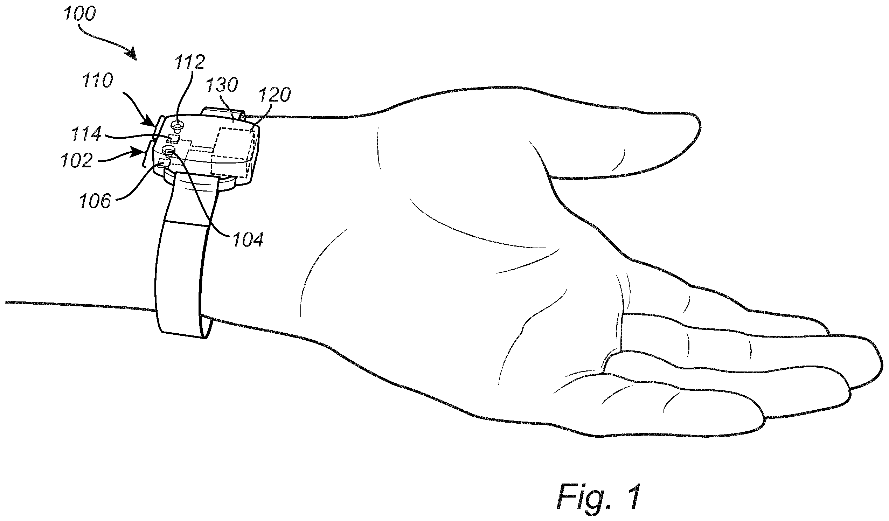

Referring now to Fig. 1, a system 100 for acquiring and processing a PPG signal will be generally described.

The system 100 may comprise a PPG detector 102. The PPG detector 102 may comprise a light source 104 and a light sensor 106 for detecting an intensity of light. The light source 104 and the light sensor 106 may be arranged on opposite sides of tissue, such as a fingertip, for acquiring an intensity of light transmitted through the tissue, so called transmittance-type PPG. However, the light source 104 and the light sensor 106 may alternatively be arranged on a common side of tissue for acquiring an intensity of light diffusely reflected by the tissue, so called reflectance-type PPG. Reflectance-type PPG may be advantageously used, because it may allow arranging the PPG detector, e.g. in a wrist-worn device, such as in a smart watch or bracelet, or in an adhesive patch which may e.g. be attached to a chest of a person.

The system 100 may further comprise a motion detector 110. The motion detector 110 may be used for acquiring a motion reference signal, which may be used for motion artifact reduction in the PPG signal acquired by the PPG detector 102.

The motion detector 110 may use any type of technology for detecting a motion artifact. The motion detector 110 may thus comprise an accelerometer or any other sensor specifically adapted to sense movements. However, as illustrated in Fig. 1, the motion detector 110 may also comprise a light source 112 and a light sensor 114 for detecting an intensity of light. The motion detector 110 may thus be configured to detect a PPG, which may be used as a motion reference signal. The motion detector 110 may use the same type of PPG measurement as used for acquiring the PPG signal, i.e. a reflectance-type PPG or transmittance-type PPG.

In one embodiment, the motion detector 110 and the PPG detector 102 may share a common light sensor 114 for detecting intensities of light based on each wavelength used by the PPG detector 102 and the motion detector 110.

The system 100 may further comprise a processing unit 120, which is configured to process the PPG signal. The processing unit 120 may be configured to process the PPG signal in order to perform motion artifact reduction and/or time domain signal reconstruction based on the acquired PPG signal. The processing performed by the processing unit 120 will be further described below.

The processing unit 120 may be arranged in a common housing 130 with the PPG detector 102 and/or the motion detector 110. This implies that the system 100 may be arranged in a single physical unit or in a few units, which may communicate with each other, through wired or wireless communication. For instance, the processing unit 120 may be provided in a common housing with one of the PPG detector 102 or the motion detector 110, whereas the other detector is provided in a separate housing and is configured to communicate an acquired signal to the processing unit 120.

The processing unit 120 may thus be arranged in a unit, which may be worn by the subject, thus allowing acquiring signals and also processing of the signals close to the subject. This may imply that the processing may be performed in real-time and analysis results may be provided directly to the subject, e.g. in a display of the housing 130.

However, the processing unit 120 may be arranged in a different unit remote from the housing 130. Thus, the housing 130 may be provided with a communication unit, for wired or wireless communication, and may transmit, possibly pre-processed, PPG signals and motion reference signals to an external unit housing the processing unit 120. The external unit may be arranged close to the subject, such as in a computer arranged in a hospital room. However, the external unit may also be very remotely placed. The communication may be over a computer network, such as the Internet, which may also allow the processing unit 120 to be arranged on any server or computer connected to the network and may hence be said to be provided "in the cloud". The processing unit 120 may then further be arranged to transmit cleaned PPG signals or extracted features back to a unit close to the subject, or to a communication unit in the housing 130, e.g. for display of results to the subject.

The processing unit 120 may be implemented in hardware, or as any combination of software and hardware. At least part of the functionality of the processing unit 120 may, for instance, be implemented as software being executed on a general-purpose computer. The system 100 may thus comprise one or more processing units, such as a central processing unit (CPU), which may execute the instructions of one or more computer programs in order to implement desired functionality.

The processing unit 120 may alternatively be implemented as firmware arranged e.g. in an embedded system, or as a specifically designed processing unit, such as an Application-Specific Integrated Circuit (ASIC) or a Field-Programmable Gate Array (FPGA).

According to an embodiment, the housing 130 may be wearable by the subject. The housing 130 may thus comprise an adhesive patch for attachment to a skin surface of the subject. The housing 130 may alternatively comprise a band element or ring-shaped element for attachment around a body part. The housing 130 could for instance comprise two band parts, which may be attached to each other in an adjustable relationship for fitting the housing 130 tightly around the body part. This may be used for arranging the housing 130 around a wrist, a finger or a torso of the subject.

Wrist-worn PPG devices such as smart watches may be used during daily ambulatory activities, which leads to relatively frequent motion artifacts. Motion in daily life can change the ambient light captured by the light sensor 106, the location of the housing 130 with respect to the wrist, and a pressure between the housing 130 and skin. All of these changes may cause fluctuations in the detected PPG signals.

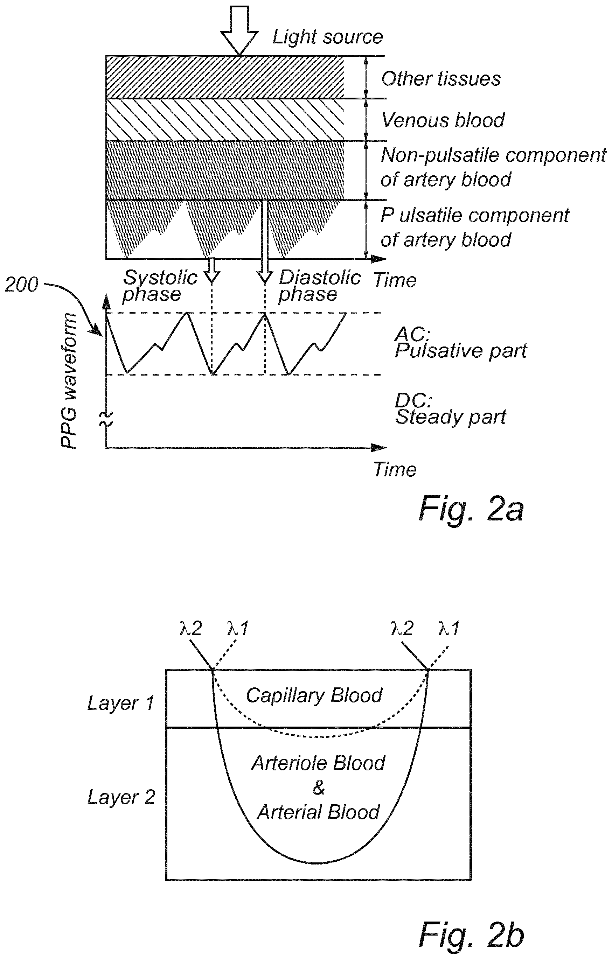

Figs 2a-b are based on figures presented in Sun Y., Thakor N., "Photoplethysmography revisited: from contact to noncontact, from point to imaging", IEEE Transactions on Biomedical Engineering, 2016, vol. 63, no. 3, pp: 463-477, and Liu J., Yan B.P.Y, Dai W.X., et al, "Multi-wavelength photoplethysmography method for skin arterial pulse extraction", Biomedical Optics Express, 2016, vol. 7, no. 10, pp: 4313-4326.

As shown in Fig. 2a, the detected PPG signal 200 can be split into two components. One component is a DC component which originates from constant absorbance by skin pigmentation, fat, muscle, bone and an average blood volume of arterial and venous blood in the illuminated tissue. The other component is an AC component which originates from the cardiac-induced variations in blood volume that are related to the cardiac rhythm (systole and diastole). The AC component may be mainly used for determining heart activity of a subject, such as for extraction of heart rate.

As illustrated in Fig. 2b, green light (indicated by a dashed line) only reaches superficial capillary bed before being reflected back to a light sensor. Red and infrared light (indicated by a solid line), however, can penetrate deeper through the skin and could reach the arteries in the subcutaneous tissue before being reflected back to a light sensor.

According to an embodiment, use is made of the fact that a PPG signal based on green light may provide a strong signal representing pulsatile component of blood, whereas a PPG signal based on infrared light may be more sensitive to motion artifacts. Thus, in one embodiment, a PPG signal may be acquired using green light, whereas a motion reference signal may be acquired using infrared light.

However, it should be realized that other wavelengths may be used. For instance, using a green or blue wavelength of light, the PPG signal is relatively insensitive to motion artifacts as light penetrates a relatively small distance into tissue. Also, using a red or infrared wavelength of light, a PPG signal representing a motion reference signal may be acquired, as the signal is relatively sensitive to motion artifacts as light penetrates a relatively large distance into tissue.

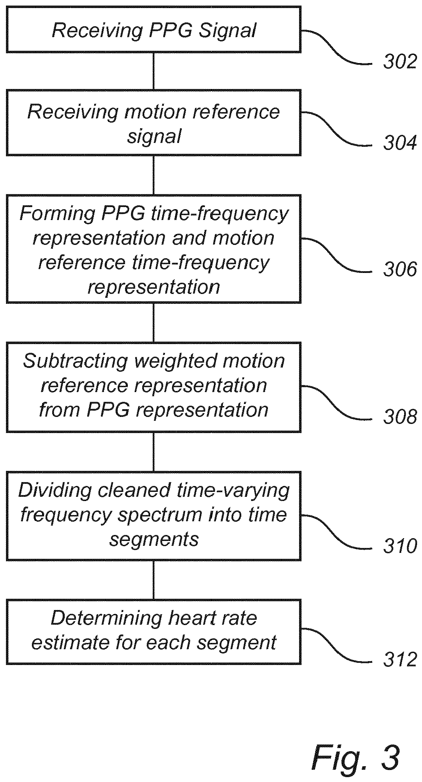

Referring now to Fig. 3, a method for motion artifact reduction will be generally described. The method may be performed by the processing unit 120.

The method comprises receiving 302 a PPG signal carrying information of heart activity of the subject. The PPG signal may be generated by the PPG detector 102 as described above. The PPG signal may or may not have been pre-processed.

The method further comprises receiving 304 a motion reference signal carrying information of a motion of the subject. The motion reference signal may be generated by the motion detector 110 as described above.

The processing unit 120 may comprise an input for receiving the PPG signal and the motion reference signal. The input may be provided from a unit within a common housing 130, such as from sensors 106, 114 or associated circuitry in the housing 130. However, the input may alternatively be provided from a communication unit which receives the signals through wired or wireless communication with the sensors 106, 114 or associated circuitry.

The method further comprises forming 306 a PPG time-frequency representation, based on the PPG signal, and a motion reference time-frequency representation, based on the motion reference signal. Thus, the PPG signal and the motion reference signal are converted to time-frequency representations, which may allow processing a time-varying frequency information of the signals.

The method further comprises subtracting 308 a weighted representation of the motion reference time-frequency representation from a weighted representation of the PPG time-frequency representation for reducing a motion artifact component in the PPG time-frequency representation and form a cleaned time-varying frequency spectrum of the PPG signal.

The motion reference signal may be more highly influenced by the motion artifact than the PPG signal. Thus, by subtracting the motion reference signal from the PPG signal, the influence of the motion artifact on the PPG signal may be reduced without need of e.g. completely removing signal in a frequency band where a motion artifact is present, which may also imply that heart activity information of the PPG signal is eliminated. This implies that influence of motion artifacts may be reduced without information of interest, e.g. heart activity information, in the PPG signal being unnecessarily removed from the PPG signal.

The method may further comprise dividing 310 the cleaned time-varying frequency spectrum into sequential time segments. Further analysis of the PPG signal may then be performed for each of the time segments so as to allow further improving the PPG signal with regard to motion artifact reduction.

The method may further comprise determining 312 an estimate of heart rate for each time segment. The estimate may be based on probability distributions of the estimated heart rate from the current time segment and the previous time segment, and confidence levels of the probability distributions. Thus, the estimated heart rate may be determined with high reliability weighing information in a current and a previous time segment based on a confidence in the information. The estimated heart rate may be used for further cleaning the signal and may be used in determining a time domain signal reconstruction of the PPG signal.

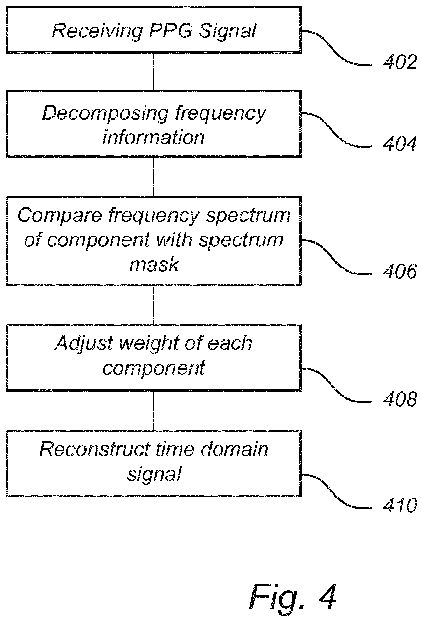

Referring now to Fig. 4, a method for time domain signal reconstruction will be generally described. The method may be performed by the processing unit 120. The method may be performed within the same processing unit, which may also be configured to perform the motion artifact reduction method as described above with regard to Fig. 3. However, it should be realized that the method may be performed as a separate process within the processing unit 120 or within a separate processing unit.

The method comprises receiving 402 a PPG signal carrying information of heart activity of the subject. The PPG signal may be a cleaned time-varying frequency spectrum signal as determined using the motion artifact reduction method described above. However, it should be realized that any type of PPG signal, in time domain, frequency domain, or time-frequency domain may be received and that the PPG signal may be a raw signal directly received from the sensor 106 or a pre-processed signal.

The method further comprises decomposing 404 frequency information of the PPG signal into a plurality of components, each component having a frequency spectrum and a weight. This implies that the frequency information may be decomposed into components having different degrees of contribution to the PPG signal. Hence, different features may be represented by different components, allowing analysis of the PPG signal. In particular, the weight of each component may indicate a magnitude of contribution of the component to the information in the PPG signal.

It should be realized that the components need not be each and every component possible to form from the information in the PPG signal. Rather, a pre-determined number of components may be formed, or only components having a weight above a threshold may be selected in forming the plurality of components based on decomposing the frequency information.

Each of the components of the plurality of components may then be further analyzed. The method thus further comprises comparing 406 the frequency spectrum of each component to a spectrum mask based on an estimate of heart rate of the subject. The spectrum mask may provide information of which frequencies are likely to represent heart activity.

The spectrum mask may be based on the estimate of the heart rate as determined in motion artifact reduction. However, the estimate of the heart rate may be formed in any other manner, even based on another type of technology for estimating heart rate.

The method further comprises adjusting 408 the weight of each component based on the comparing. Thus, the importance of the components in representing heart activity may be updated, such that components which mainly represent motion artifact frequencies or noise frequencies may be given a lower weight.

The adjusting of the weight may comprise selecting the components with highest weights after updating weights based on the comparing, such that components for which a low weight is determined the weight may be set to zero. The adjusting of the weight may also comprise applying a sigmoid function to the weights after updating the weights based on the comparing such that components having high weights may be given an even higher influence than components having low weights.

The method further comprises reconstructing 410 a time domain signal based on recombination of the plurality of components with adjusted weights. The reconstruction is thus based on updated weights of the components representing importance of the components.

This implies that a reliable and robust manner of forming a time domain signal representation of the PPG signal may be provided, while reducing influence of noise or motion artifacts in the time domain representation.

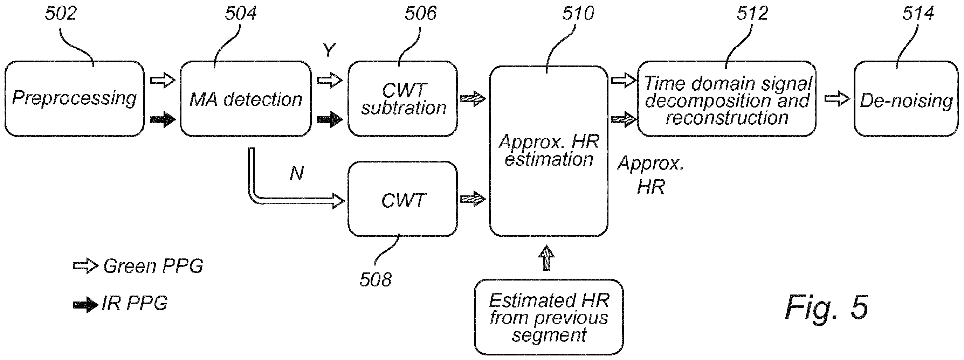

Referring now to Figs. 5-10, a detailed description of an embodiment for motion artifact reduction and time domain signal reconstruction of a PPG signal will be described. It should be realized that individual features of this embodiment may be combined in many other ways and, not necessarily with the other features described in this embodiment.

In Fig. 5, a flow chart indicating processing steps is shown. In this processing algorithm, a detected signal from an infrared (IR) PPG channel is taken as the motion reference signal, whereas a detected signal from a green PPG channel is taken as the PPG signal for heart rate (HR) extraction.

The ratio between heart activity signal information and motion artifact (MA) information is different for recorded PPG signals by green and IR light. The IR PPG signal contains more MA information and may therefore be suitable as a motion reference signal.

The frequency components of MA in recorded PPG by green and IR light are similar in periodic motion situation. The frequency components of MA in recorded PPG by green and IR light may also be similar in non-periodic motions. This implies that the IR PPG is advantageously used to represent MA content in the green PPG signal.

Further, the HR variation between 4s segments may be assumed to obey a normal distribution expressed as $X \sim N(0; 0.1)$. Thus, the HR variation $X$ is represented by a normal distribution N around 0, with a standard deviation of 0.1 Hz. This may be used as an assumption which is generally applicable to any subject. However, according to another embodiment, a distribution of the HR variation may be determined for a specific subject during set-up of the system and the determined distribution may then be used in the HR estimation, as described below.

The processing of the PPG signal and the motion reference signal starts with preprocessing 502 by bandpass filtering. Then, detection 504 of presence of motion artifact is performed based on the input PPG signal and motion reference signal. This step uses peak to average ratio from the green channel and power ratio between the green and IR PPG channels as input parameters for a classifier. A result of classification will decide whether a subtraction operation between green and IR signal will be performed or not. It should be realized that presence of MA may be determined in other ways.

If motion artifact is present, motion artifact reduction (MAR) is performed 506 by spectrum subtraction after continuous wavelet transform (CWT) of the PPG signal and the motion reference signal, resulting in a time-varying signal spectrum. In case of no motion artifact, the CWT of the green PPG signal is determined 508 and will form the time-varying signal spectrum.

After that, the HR is estimated 510 taking into consideration both the HR from a previous time segment and the spectrum from a current time segment. Approximate entropy and peak to average ratio may be used to decide a confidence level of the HR estimate for the previous time segment and the HR estimate for the current segment. Thus, the HR estimation may provide frequency tracking of the HR.

Then, time domain signal decomposition and reconstruction may be performed 512. Here, a frequency mask based on the HR estimated from the HR estimation 510 is obtained for signal reconstruction. This frequency mask is then compared with each component after singular spectrum analysis (SSA) decomposition of the PPG signal to decide the weights of the components. The PPG signal can be reconstructed by combining those components.

Finally, de-noising 514 may be applied by time domain processing.

The above-described steps are discussed in more detail below.

In preprocessing 502 of the signals, to cancel an effect of light intensity, the signal is divided by its DC component that is proportional to the light intensity. The DC component may be calculated by means of recorded PPG signals of green and IR light during a stationary situation.

HR of subjects may vary within a range set by 24 to 240 beats per minute. In preprocessing 502 of the signals, both the green and IR signals are filtered with a second order infinite impulse response (IIR) bandpass filter (0.4-4Hz) to remove the noise and MA components outside a frequency band of interest. After the preprocessing, only in-band MA will remain together with the PPG signal.

The detection 504 of presence of MA may be useful to avoid subtraction of the motion reference signal from the PPG signal when no MA is present.

When no MA is present, the main components in both green and IR PPG signals are clean PPG signals. The subtraction in MAR 506 may therefore lead to cancelling the HR component itself. Therefore, in situations of no or small MA, the CWT result of the green channel (without MAR by subtracting the IR from it) may be taken to form the time-varying signal spectrum, providing HR information of the current time segment.

The motion detection may be implemented by a support vector machine (SVM), based on two parameters:

    1. The peak to noise ratio of green PPG signal in frequency domain.
    2. The power ratio between green PPG to IR PPG signals.

The peak to noise ratio of green PPG signal is much higher in situations of no or small MA or in periodical motion situations than in other motion situations. This is because in the first two situations, the PPG signal has a clear HR component while in the latter, the MA component can spread over frequency spectra, leading to an increase of noise.

The power ratio between green PPG to IR PPG is another indicator. Usually in no MA situation, the signal from green PPG channel (AC/DC) may be more than 10 times higher than the signal from IR PPG channel. While in motion situations, the MA component in IR may be much larger than the signal in green channel.

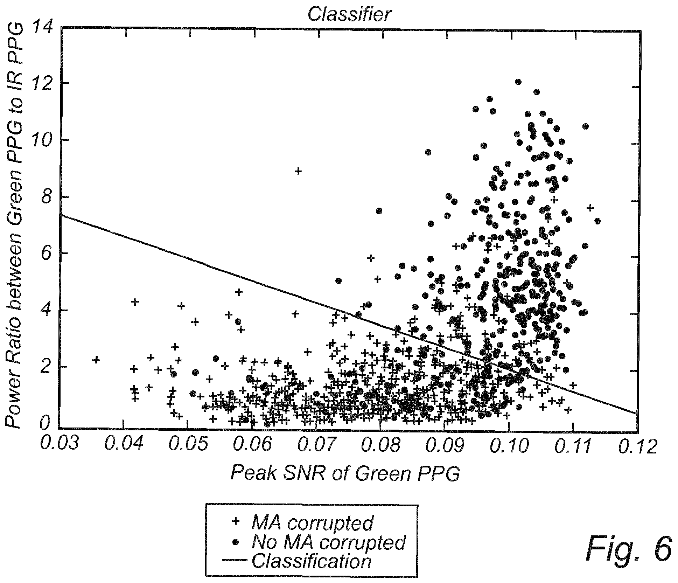

Therefore, a higher peak to noise ratio and a higher power ratio between green and IR signals tend to give a no motion decision while lower value of both parameters tend to give a motion decision. An example of a separation of the two classes by the classifier is shown in Fig. 6 for a dataset of three subjects.

The MA in IR PPG may have good frequency similarity with the MA in green PPG in periodic and non-periodic motion situations. Moreover, the ratio of MA to PPG signal in IR PPG is much higher than in green PPG. Therefore, if a scaled IR signal is subtracted from the green signal in frequency domain, the MA component can be effectively removed and the HR component will be enhanced.

PPG signal is a quasi-periodical signal. Therefore, a signal transformation having both frequency and time domain information is advantageous. Thus, a continuous wavelet transform (CWT) of the PPG signal and the motion reference signal may be used. Compared to short time Fourier transform (STFT), which has high accuracy either in time domain or frequency domain according to the window size, CWT may be better for time-frequency analysis since it uses the size-adjustable window. However, it should be realized that, in some embodiments, STFT may be used or another transform providing a time-frequency representation may be used.

In spectrum domain, the spectrum of corrupted PPG signal $P_m(f)$ can be expressed as set out in Eq. (1) below, where $P_c(f)$ is the clean PPG signal component and $MA(f)$ is the motion artifact component:

$$P_m(f) = P_c(f) + MA(f) \qquad\qquad \text{Eq. (1)}$$

The clean PPG signal and MA signal may be assumed to be uncorrelated. The frequency domain MA removal can be obtained as expressed in Eq. (2), where a scaling factor $W$ is decided by using an adaptive filter (e.g. a wiener filter) to obtain minimum residue motion component after removal.

$$P_c(f) = P_{cG}(f) + MA_G(f) - W * \left( P_{cIR}(f) + MA_{IR}(f) \right) \qquad \text{Eq. (2)}$$

Here, $P_{cG}(f)$ is the clean PPG signal component in the green PPG and $MA_G(f)$ is the motion artifact component in the green PPG, whereas $P_{cIR}(f)$ is the clean PPG signal component in the IR PPG and $MA_{IR}(f)$ is the motion artifact component in the IR PPG. The $MA_G$ and $MA_{IR}$ components have similar frequency components and the $P_{cIR}(f)$ component is very small in comparison to $MA_{IR}(f)$. Thus, the main HR component $P_{cG}(f)$ can remain intact after motion removal by means of the subtraction in Eq. (2).

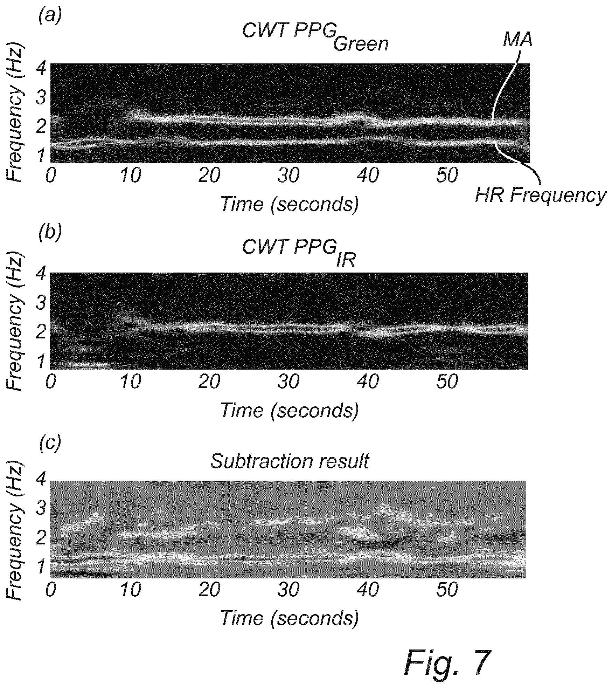

An example of periodic motion (wrist moving) removal is shown in Fig. 7 in terms of CWT representation of the signal. Comparing the time-frequency representation in graph (a) and graph (b) of Fig. 7, it can be observed that the HR component in the green PPG signal is much larger than the HR component in the IR PPG signal and the MA component in IR PPG is indeed very similar to the MA component in the green PPG. After motion removal, the MA component is cancelled and the HR component remains as shown in part (c) of Fig. 7.

However, in a no motion situation, there will be both clean PPG signals in IR and green channel, and the HR component can be cancelled by itself if the subtraction operation is performed. This is the reason why the aforementioned motion detection classifier may be advantageous. If the time segment is classified as being a no MA situation, the spectrum of green PPG signal is chosen instead of doing spectrum subtraction.

After determination of a clean time-varying signal spectrum, an estimation 510 of HR may be performed and used for improving the acquired PPG signal. The approximate HR of the current time segment is estimated based on both the time-varying signal spectrum of the current time segment and the HR frequency estimated in the previous time segment. Further, confidence levels of both the approximate HR of the current time segment and the approximate HR of the previous time segment are also used for the HR frequency decision.

The output of a time-varying signal spectrum is a sequence of frequency spectra, each corresponding to a time segment. The time segment may for instance be a 4s time segment. The frequency spectrum for a time segment may comprise only the HR related components of PPG, thanks to reduction of motion artifacts from the signal.

The amplitude information of the frequency spectrum is used to obtain a probability distribution of HR of a current time segment. There may, for instance, be 256 components in total in the frequency spectrum (from 0.5Hz to 4Hz). The number of components depend on the resolution used in determination of the time-frequency representation of the PPG signal.

The sum of the components in the frequency spectrum may be normalized to 1 by scaling each component with the same factor. This implies that a probability distribution of HR of the current time segment is given by the normalized amplitude information of the frequency spectrum.

The HR frequency estimated in the previous time segment may also be used to predict the HR in the current segment. The HR frequency for the current time segment may be based on the HR frequency of the previous time segment in combination with a probability of HR variation. Thus, the HR frequency of the current time segment may be determined as a normal distribution of $X \sim N(HR_{est}; 0.1)$. Thus, the probability distribution of HR frequency of the current time segment may be given as a normal distribution around the HR frequency of the previous time segment, $HR_{est}$, with a standard deviation of 0.1 Hz. Thus, two probability distribution functions for HR frequency estimation are available.

The confidence levels of these two distribution functions may, in one embodiment, be decided by two parameters: the approximate entropy of the signal and the peak-to-noise ratio calculated in frequency domain.

The approximate entropy may quantify the amount of regularity and the random fluctuations in time domain signals. When the signal contains only clean PPG signal with high regularity, a low entropy can be obtained. While signals with more non-periodical MA components will show a higher entropy. Therefore, the signal segments with a low entropy and a high peak-to-noise ratio can be recognized as segments which have high confidence level and thus give a true or reliable HR frequency estimation.

Thus, the two probabilities: $P(Pre_{fj} == \text{True})$ for the estimated HR at frequency $fj$ from previous time segment $Pre_{fj}$ and $P(Spec == \text{True})$ for the HR distribution obtained from the current time segment $Spec$ are available. These two probabilities may be assumed to be independent.

In Bayesian inference, it is desired to determine the probability that a hypothesis HR frequency $fi$ of the current time segment $Cur_{fj}$ is true given the existence of evidence $Pre_{fj}$ (the HR frequency estimated from the previous segment) and $Spec$ (the HR frequency estimated from the spectrum of current segment) as expressed in Eq. (3).

In Eq. (3), the probability $P(Cur_{fi}|Pre_{fj} == True)$ obeys normal distribution with mean of $fj$, and the probability $P(Cur_{fi}|Spec == True)$ is decided by its spectrum (result of CWT) as discussed above. Further, the probability $P(Cur_{fi}|Pre_{fj} == False)$ and the probability $P(Cur_{fi}|Spec == False)$ is equal to $1/m$, where $m$ is the number of frequency components or candidates $fi$ in the frequency spectrum (m=256 in the present embodiment). Together with the probabilities $P(Pre_{fj} == \text{True})$, $P(Spec == \text{True})$, $P(Pre_{fj} == \text{False})$, and $P(Spec == \text{False})$, a HR frequency with a highest probability may be obtained. This approximately estimated HR frequency may then be used in signal reconstruction in order to remove MA from signal reconstruction. Also, the approximately estimated HR frequency may be used as a reliable estimate of the HR of the current time segment.

$$P\left(Cur_{fi}\middle|Pre_{fj},Spec\right) = \frac{P\left(Pre_{fj},Spec,Cur_{fi}\right)}{P\left(Spec,Cur_{fi}\right)}$$

$$\propto P\left(Pre_{fj} == True, Spec == True, Cur_{fi}\right) +$$
$$P\left(Pre_{fj} == True, Spec == False, Cur_{fi}\right) + \qquad \text{Eq. (3)}$$
$$P\left(Pre_{fj} == False, Spec == True, Cur_{fi}\right) +$$
$$P\left(Pre_{fj} == False, Spec == False, Cur_{fi}\right)$$

It may be noted that the resulting approximate HR can be estimated mainly from the previous time segment when the confidence level of the current time segment is low, probably because the MAR is not working well. Likewise, the HR can also be estimated mainly from the current time segment when the confidence level of the previous time segment is low. This may occur when a motion stops and the current time segment is recognized as a no MA situation or the frequency spectrum of the current time segment is more regular than the frequency spectrum of the previous time segment and, thus, the current time segment has a high confidence level.

The estimation of HR frequency improves a robustness of determination of heart activity and may also be used in improving a time domain signal reconstruction, as will now be described.

The HR usually shows small variations from beat to beat. Using HR detection in the frequency domain provides only an average HR within a segment. Analysis in the time domain could provide the peak positions that can be used to obtain beat to beat HR. Thus, reconstruction of the time domain PPG signal may allow further analysis of the heart activity.

There are two steps in the time domain signal reconstruction described below. Firstly, the time domain signal of a single time segment is decomposed into a group of oscillatory and noise components by singular spectrum analysis (SSA), providing each component with its own weight.

Secondly, a frequency mask is then used to scale these weights.

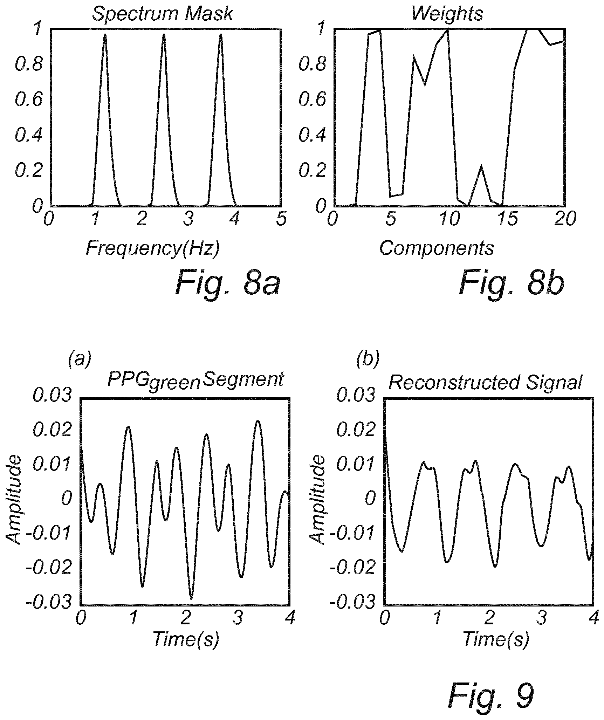

Based on the estimated approximate HR frequency, a spectrum mask can be obtained as shown in Fig. 8a. The frequency mask may be a composition of three normal distributions around the fundamental, the second and the third harmonics of the HR frequency. The normal distributions may thus provide a non-zero probability of a frequency relating to HR frequency in three ranges given by $\{f_{HR} - \Delta, f_{HR} + \Delta\}$, $\{2 * f_{HR} - \Delta, 2 * f_{HR} + \Delta\}$, and $\{3 * f_{HR} - \Delta, 3 * f_{HR} + \Delta\}$, where $\Delta$ is based on the standard deviation $\sigma = 0.1$Hz. This distribution reflects the probability of a frequency to be part of the clean PPG signal.

The weight of each component from SSA is decided by the correlation between a frequency spectrum of the component and the frequency spectrum mask. Thus, the weight of the component, as determined above from the SSA, may be updated based on the correlation between the frequency spectrum of the component and the frequency spectrum mask. One example of resulting weights is shown in Fig. 8b, where the weight of components 1-20 from the SSA are indicated.

The updated weights may be re-scaled to a range between 0 and 1, as indicated in Fig. 8b. Also and or alternatively, weights may be scaled based on a threshold or soft threshold, such as based on a Sigmoid function, for pushing the weights towards either 0 or 1, such that mainly the components that are well correlated with the frequency spectrum mask will be taken into account in reconstruction of the time domain signal.

It should also be realized that information of motion artifacts may be used in updating of the weights. Hence, information of frequency content of motion artifacts may also be used in relation to the components of the SSA in order to lower a weight of a component that comprises frequencies determined to be related to motion artifacts. MA frequencies may be determined based on the motion reference signal, such as the IR PPG signal.

When the weights of the components of SSA have been updated, a reconstruction of a time domain signal for the time segment may be formed based on the SSA components.

The reconstruction may be performed for every time segment of 4 seconds. Fig. 9 shows one example of the original green PPG signal in part (a) and the reconstructed signal in part (b), which shows a clear HR component.

After reconstruction of the time domain signal, de-noising may be applied. The de-noising may be applied over a time period longer a time segment. Thus, for instance, the de-noising may be applied for an 8 second time period spanning two consecutive 4 second time segments.

The de-noising may operate to remove discontinuities, which may be due to the time domain signal reconstruction being based on previous steps in a 4s non-overlapping window.

The de-noising may be performed as a SSA operation to remove the noise from the signal by identifying major frequency components in the time period, allowing noise to be removed.

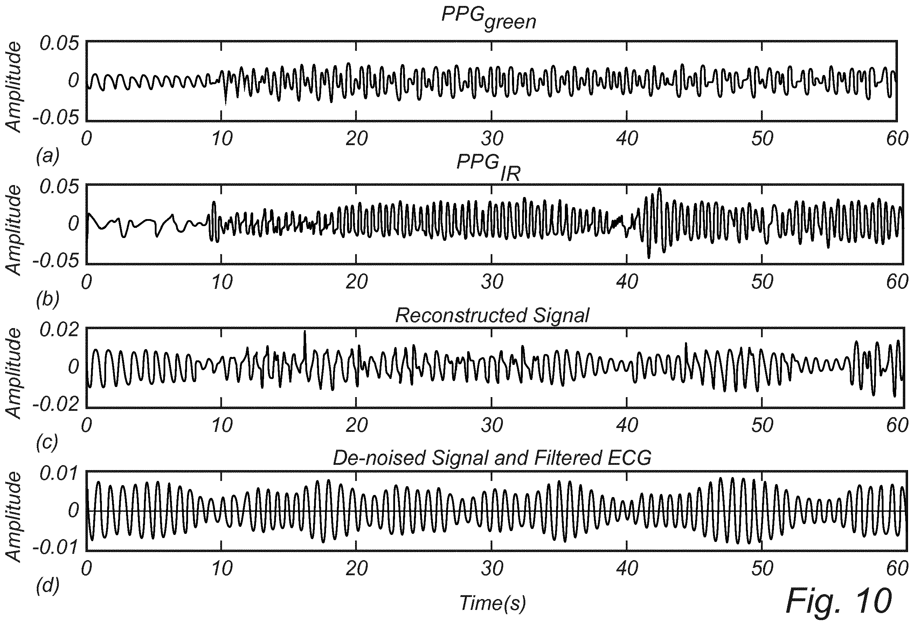

The de-noising could also apply the information from a time segment in the reconstructed time domain signal information to nearby time segments. An example is illustrated in Fig. 10, showing a green PPG signal in part (a), an IR PPG signal in part (b), a reconstructed time domain signal in part (c) and a de-noised signal in part (d). As is shown in part (c) of Fig. 10, for the period of 55-60s, the reconstruction step is not ideal, However, by means of de-noising, it is still possible to extract the clean PPG signal in this time segment with the help of previous time segment information.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For instance, the decomposition of frequency information need not necessarily use SSA. Although SSA may be more efficient in finding relevant components, other decomposition algorithms may be used, such as independent component analysis (ICA), principle component analysis (PCA), or empirical model decomposition (EMD).

**Claims**

1. A method for time domain signal reconstruction for representing heart activity of a subject from a photoplethysmogram, PPG, signal, said method comprising:

   receiving (402) a PPG signal carrying information of heart activity of the subject;
   decomposing (404) frequency information of the PPG signal into a plurality of components, each component having a frequency spectrum and a weight;
   for each component of the plurality of components:

   comparing (406) the frequency spectrum of the component to a spectrum mask based on an estimate of heart rate of the subject; and
   adjusting (408) the weight of the component based on the comparing; and

   reconstructing (410) a time domain signal based on recombination of the plurality of components with adjusted weights.

2. The method according to claim 1, further comprising determining the spectrum mask based on the estimate of heart rate of the subject.

3. The method according to claim 1 or 2, wherein the spectrum mask comprises normal distributions around the estimate of the heart rate and one or more harmonics of the estimate of the heart rate, the spectrum mask representing probabilities of frequencies corresponding to heart rate information of the PPG signal based on the normal distributions.

4. The method according to claim 3, wherein said comparing (406) comprises correlating the frequency spectrum of the component with the spectrum mask representing probabilities of frequencies corresponding to heart rate information of the PPG signal for scaling the weight of the component.

5. The method according to any one of the preceding claims, wherein the decomposing (404) frequency information of the PPG signal is performed for a plurality of sequential time segments to form a plurality of time-series components, and wherein the decomposing frequency information of the PPG signal into the plurality of time-series components comprises performing singular spectrum analysis, SSA, of the PPG signal.

6. The method according to claim 5, further comprising calculating a Fourier transform of a component for determining the frequency spectrum of the component.

7. The method according to any one of the preceding claims, further comprising performing (308) motion artifact reduction on the PPG signal to form a cleaned PPG signal, wherein decomposing (406) frequency information of the PPG signal into the plurality of components is based on the cleaned PPG signal.

8. The method according to any one of the preceding claims, further comprising determining (312) the estimate of heart rate based on the received PPG signal.

9. The method according to any one of the preceding claims, further comprising dividing the received PPG signal into sequential time segments, wherein the decomposing, comparing, adjusting and reconstructing is performed on the PPG signal within a time segment.

10. The method according to claim 9, further comprising analyzing a reconstructed time domain signal spanning more than a single time segment for reducing noise in the reconstructed time domain signal.

11. Use of the time domain signal reconstruction formed by the method according to any one of preceding claims for monitoring heart rate variability.

12. A computer program product or a computer-readable medium storing computer-readable instructions such that when executed on a processing unit the computer program product will cause the processing unit to perform the method according to any one of claims 1-10.

13. A system (100) for time domain signal reconstruction for representing heart activity of a subject from a photoplethysmogram, PPG, signal, said system (100) comprising:

a processing unit (120), said processing unit (120) being configured to:

receive (402) a PPG signal carrying information of heart activity of the subject;
decompose (404) frequency information of the PPG signal into a plurality of components, each component having a frequency spectrum and a weight;
for each component of the plurality of components:

compare (406) the frequency spectrum of the component to a spectrum mask based on an estimate of heart rate of the subject; and
adjust (408) the weight of the component based on the comparing; and

reconstruct (410) a time domain signal based on recombination of the plurality of components with adjusted weights.

14. The system according to claim 13, further comprising a PPG detector (102) for generating the PPG signal.

15. The system according to claim 14, further comprising a motion detector (110) for generating a motion reference signal for use in motion artifact reduction of the PPG signal.

Fig. 1

Fig. 2a

Fig. 2b

```
┌─────────────────────────────┐
│   Receiving PPG Signal       │────── 302
└─────────────────────────────┘
              │
┌─────────────────────────────┐
│   Receiving motion reference │
│   signal                     │────── 304
└─────────────────────────────┘
              │
┌─────────────────────────────┐
│  Forming PPG time-frequency  │
│  representation and motion   │
│  reference time-frequency    │────── 306
│  representation              │
└─────────────────────────────┘
              │
┌─────────────────────────────┐
│  Subtracting weighted motion │
│  reference representation    │
│  from PPG representation     │────── 308
└─────────────────────────────┘
              │
┌─────────────────────────────┐
│  Dividing cleaned time-varying│
│  frequency spectrum into time│
│  segments                    │────── 310
└─────────────────────────────┘
              │
┌─────────────────────────────┐
│  Determining heart rate      │
│  estimate for each segment   │────── 312
└─────────────────────────────┘
```

*Fig. 3*

Fig. 4

502 Preprocessing → 504 MA detection → Y → 506 CWT subtration → 510 Approx. HR estimation → Approx. HR → 512 Time domain signal decomposition and reconstruction → 514 De-noising

N → 508 CWT →

510 Approx. HR estimation

Estimated HR from previous segment

⇨ Green PPG

➡ IR PPG

Fig. 5

EP 3 501 381 A1

19

Fig. 6

*Fig. 7*

Spectrum Mask

Weights

## Fig. 8a

## Fig. 8b

(a)

PPG$_{green}$ Segment

(b)

Reconstructed Signal

## Fig. 9

*PPG_green*

(a)

*PPG_IR*

(b)

Reconstructed Signal

(c)

De-noised Signal and Filtered ECG

(d)

Time(s)

*Fig. 10*

Europäisches
Patentamt

European
Patent Office

Office européen
des brevets

# EUROPEAN SEARCH REPORT

Application Number

EP 17 21 0372

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/180986 A1 (KONINKL PHILIPS NV [NL]) 3 December 2015 (2015-12-03)<br>* page 3, lines 11, 18, 19 *<br>* page 6, lines 18, 27, 28 *<br>* page 7, line 16 *<br>* page 11, line 24 *<br>* page 18, lines 5, 9-12, 15, 20 *<br>* page 19, lines 2, 27, 29, 30 *<br>* page 20, lines 2, 4-6 *<br>* page 21, lines 23, 25, 28 *<br>* figure 8 * | 1-15 | INV.<br>A61B5/00<br>A61B5/024 |
| A | WO 2015/130929 A2 (WORCESTER POLYTECH INST [US]; CHON KI H [US]; CHONG JOWOON [US]; MENDE) 3 September 2015 (2015-09-03)<br>* the whole document * | 1-15 | |
| A | ZI-HAO ZHANG ET AL: "A new framework to extract heart rate information from photoplethysmographic (PPG) signals with strong motion artifacts",<br>2013 IEEE INTERNATIONAL CONFERENCE OF IEEE REGION 10 (TENCON 2013), IEEE,<br>1 November 2015 (2015-11-01), pages 1-4,<br>XP032845216,<br>ISSN: 2159-3442, DOI:<br>10.1109/TENCON.2015.7373104<br>ISBN: 978-1-4799-2825-5<br>[retrieved on 2016-01-05]<br>* the whole document * | 1-15 | TECHNICAL FIELDS SEARCHED (IPC)<br><br>A61B |

The present search report has been drawn up for all claims

| Place of search | Date of completion of the search | Examiner |
|---|---|---|
| The Hague | 31 May 2018 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 21 0372

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2018

| Patent document cited in search report | | Publication date | Patent family member(s) | | Publication date |
|---|---|---|---|---|---|
| WO 2015180986 | A1 | 03-12-2015 | CN | 106413530 A | 15-02-2017 |
| | | | EP | 3148404 A1 | 05-04-2017 |
| | | | JP | 2017519548 A | 20-07-2017 |
| | | | US | 2017071547 A1 | 16-03-2017 |
| | | | WO | 2015180986 A1 | 03-12-2015 |
| WO 2015130929 | A2 | 03-09-2015 | US | 2016367198 A1 | 22-12-2016 |
| | | | WO | 2015130929 A2 | 03-09-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SUN Y. ; THAKOR N.** Photoplethysmography revisited: from contact to noncontact, from point to imaging. *IEEE Transactions on Biomedical Engineering,* 2016, vol. 63 (3), 463-477

- **LIU J. ; YAN B.P.Y ; DAI W.X. et al.** Multi-wavelength photoplethysmography method for skin arterial pulse extraction. *Biomedical Optics Express,* 2016, vol. 7 (10), 4313-4326